# EUROPEAN PATENT APPLICATION

(11) **EP 1 389 638 A1**
(43) Date of publication of application: **18.02.2004**
(21) Application number: 02017629.3
(22) Date of filing: 06.08.2002
(51) Int. Cl.: C12Q 1/68

(54) **Improved fluorescent resonance energy transfer probes**

(71) Applicant: Roche Diagnostics GmbH, 68305 Mannheim (DE); F.HOFFMANN-LA ROCHE AG, 4070 Basel (CH)
(72) Inventor: The designation of the inventor has not yet been filed

(57) **Abstract**

The invention is directed to a pair of FRET hybridization probes hybridizing adjacently to a target nucleic acid sequence, each hybridization probe comprising (i) a nucleotide sequence entity which is substantially complementary to the sequence of the target nucleic acid, (ii) a fluorescent entity, said entity being either the FRET donor entity or the FRET acceptor entity, and (iii) a spacer entity connecting said nucleotide sequence entity and said fluorescent entity. According to one aspect of the invention, the intensity of fluorescence emission from the FRET donor entity and the intensity of fluorescence emission from the FRET acceptor entity is not substantially affected by any quenching activity of nucleotide residues either present in the sequence of the target nucleic acid or present in said nucleotide sequence entities of the hybridization probes.

## Description

The invention originates from the field of Real Time PCR. More specifically, the invention is directed to an improved design of FRET Hybridization Probes.

### Prior art Background

In kinetic real-time PCR, the formation of PCR products is monitored in each cycle of the PCR. The amplification is usually measured in thermocyclers which have additional devices for measuring fluorescence signals during the amplification reaction. A typical example of this is the Roche Diagnostics LightCycler (Cat. No. 20110468). The amplification products are for example detected by means of fluorescent labeled hybridization probes which only emit fluorescence signals when they are bound to the target nucleic acid or in certain cases also by means of fluorescent dyes that bind to double-stranded DNA. A defined signal threshold is determined for all reactions to be analysed and the number of cycles Cp required to reach this threshold value is determined for the target nucleic acid as well as for the reference nucleic acids such as the standard or housekeeping gene. The absolute or relative copy numbers of the target molecule can be determined on the basis of the Cp values obtained for the target nucleic acid and the reference nucleic acid (Roche Diagnostics LightCycler operator manual(Cat. No. 2 0110468))

The FRET hybridization probe test format, which may be used in real time PCR, is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively, fluorescence decrease of the FRET donor component may be monitored. Among all detection formats known in the art, this FRET-hybridization probe format has been proven to be highly sensitive, exact and reliable (WO 97/46707; WO 97/46712; WO 97/46714. The design of appropriate FRET hybridization probe sequences may sometimes be limited by the special characteristics of the target nucleic acid sequence to be detected.

Alternatively, it is also possible to use a fluorescent-labeled primer and only one labeled oligonucleotide probe (Bernard, P. S., et al., Anal Biochem 255 (1998) 101-7.).

Another application of FRET hybridization probes is melting curve analysis. In such an assay, the target nucleic acid is amplified first in a typical PCR reaction with suitable amplification primers The hybridization probes may already be present during the amplification reaction or added subsequently. After completion of the PCR-reaction, the temperature of the sample is constitutively increased, and fluorescence is detected as long as the hybridization probe was bound to the target DNA. At melting temperature, the hybridization probes are released from their target, and the fluorescent signal is decreasing immediately down to the background level. This decrease is monitored with an appropriate fluorescence versus temperature-time plot such that a first derivative value can be determined, at which the maximum of fluorescence decrease is observed.

However, for kinetic real time PCR as well as for melting curve analysis, tremendous differences in absolute signal intensities have been observed for different pairs of FRET hybridization probes, although being labeled with the same couple of fluorescent dyes. Moreover, this phenomenon is independent from the couple of fluorescent dyes which is used.

The reason for the observed effect is unknown, although one may speculate that it could be due to quenching or dequenching effects of G residues which have been disclosed previously in various systems (WO 01/36668, Seidel, C. A. M., et al., J Phys Chem 100 (1996) 5541-53, 1996).

In most cases, G residues causing quenching effects are usually located in close spatial vicinity to the respective fluorescent compound (EP 1 046 717). Moreover, based on this effect, it is was possible in some cases to set up an assay, wherein fluorescent emission of an unhybridized labeled probe is quenched by internal residues and hybridization can monitored due to a dequenching effect occuring as soon as the probe is being hybridized to a complementary target sequence (WO 01/73118).

Due to the unpredictable overall structures of nucleic acids, however, G residues causing quenching effects can be located at different positions in the target DNA as well as in the hybridzation probes themselves.

In any case, the observed effect is highly disadvantageous especially with respect to the design of multiplex assays, characterized in that within one reaction vessel, one or more target sequences are amplified and quantitatively analyzed with two or multiple pairs of FRET hybridization probes. Thus there is a need in the art for an improved design of FRET Hybridization probes wherein the absolute signal is not affected by the base composition and sequence of the target nucleic acid.

### Brief description of the invention

This problem is resolved by FRET hybridization probes according to the present invention.

It is directed to a pair of FRET hybridization probes, wherein the emission signals of the fluorescent moieties are not interfered by any quenching activity of any nucleotide residue in the vicinity of said fluorescent moieties.

More precisely, the invention is directed to a pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity, and
- a spacer entity connecting said nucleotide sequence entity and said fluorescent entity,
wherein said spacer entities of the two members of said pair of FRET hybridization probes are capable of forming non covalent interactions with each other. The non covalent interactions may be, for example, nucleotide base pairing interactions and preferably A/T base pairing interactions.

Defined in another way, the invention is directed to a pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity.

As will be shown in the examples, due to the presence of the spacer entities, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probes themselves.

### Description of the Figures

### Figure 1

Fluorescence versus cycle number plot of the real time PCR experiment disclosed in example 2 using Fluorescein/JA286 FRET hybridization probes to detect a FactorV amplicon.
+ Stem: FRET hybridization probes carrying a stem according to the invention
- Stem: FRET hybridization probes without stem.
a) 10⁶ copies b) 10⁴ copies of target DNA

### Figure 2

1^{st} derivative of fluorescence versus temperature plot showing the melting curve analysis of the experiment disclosed in example 3 using Fluorescein/JA286 FRET hybridization probes.
+ Stem: FRET hybridization probes carrying a stem according to the invention
- Stem: FRET hybridization probes without stem.
a) 10⁶ copies b) 10⁴ copies of Target DNA

### Figure 3

Fluorescence versus cycle number plot of the real time PCR experiment disclosed in example 4 using Fluorescein/LC-Red-640 FRET hybridization probes to detect a Factor V amplicon.
+ Stem: FRET hybridization probes carrying a stem according to the invention
- Stem: FRET hybridization probes without stem.
a) 10⁶ copies b) 10⁴ copies of Target DNA

### Figure 4

1^{st} derivative of fluorescence versus temperature plot showing the melting curve analysis of the experiment disclosed in example 5 using Fluorescein/LC-Red-640 FRET hybridization probes
+ Stem: FRET hybridization probes carrying a stem according to the invention
- Stem: FRET hybridization probes without stem.
a) 10⁶ copies b) 10⁴ copies of Target DNA

### Figure 5

Fluorescence versus cycle number plot of the real time PCR experiment disclosed in example 6 using Fluorescein/JA286 FRET hybridization probes to detect a G6PDH amplicon.
+ Stem: FRET hybridization probes carrying a stem according to the invention
- Stem: FRET hybridization probes without stem
a) 10⁸ copies b) 10³ copies of Target DNA

### Figure 6

Fluorescence versus cycle number plot of the real time PCR experiment disclosed in example 7 using Fluorescein/JA286 FRET hybridization probes to detect a FactorV amplicon comprising different A/T stems.
- without: FRET hybridization probes carrying no stem
- 1A/T: FRET hybridization probes carrying a 1 base pair A/T stem
- 3 A/T: FRET hybridization probes carrying a 3 base pair A/T stem
- 5 A/T: FRET hybridization probes carrying a 5 base pair A/T stem

### Figure 7

1^{st} derivative of fluorescence versus temperature plot showing the melting curve analysis of the experiment disclosed in example 8 using Fluorescein/JA286 FRET hybridization probes comprising different A/T stems
- without: FRET hybridization probes carrying no stem
- 1A/T: FRET hybridization probes carrying a 1 base pair A/T stem
- 3 A/T: FRET hybridization probes carrying a 3 base pair A/T stem
- 5 A/T: FRET hybridization probes carrying a 5 base pair A/T stem

### Detailed description of the invention

As it is known in the art, the FRET hybridization probe test format is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.

According to the invention, the desired effect of preventing any quenching effect is achieved by a pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein said spacer entities of the two members of said pair of FRET hybridization probes are capable of forming non covalent interactions with each other.

In addition to the desired effect of prevention of quenching effects, such an inventive pair of FRET hybridization probes has further advantages. First, the kinetics of formation of the ternary complex composed of the target nucleic acid and the two members of the pair of hybridization probes may be accelerated. Second, the melting temperature of the FRET hybridization probes in some instances may be altered at least slightly. In certain cases, such an adjustment may be required for an optimal design of a multiplex assay.

In the context of the present invention, the term "spacer entity" is a chemical linker structure with a molecular weight of at least 300.

In a preferred embodiment, said spacer entities are nucleotide residues characterized in that the additional residues of the first hybridization probe may form base pairing interactions with the nucleotide residues of the second hybridization probe thus forming a stem structure, when hybridized to the target nucleic acid.

In contrast to standard FRET hybridization probes known in the art which are characterized in that the fluorescent compounds are in close proximity to the nucleotides forming the probe/target hybridization complex, the design of FRET hybridization probes according to the invention avoids any potential interference of the fluorescent compounds with e.g. G residues that are present within the probe/target hybridization complex.

In the context of the present invention, the term "substantially complementary" shall mean, that the respective sequences specifically hybridize to each other under standard annealing conditions. The length of the nucleotide sequence entity which is substantially complementary to the sequence of the target nucleic acid may vary between 10 and 40 nucleotide residues. Preferably, but depending on the AT content of the target nucleic acid, said length is between 15 and 30 nucleotide residues. At least, a perfect Watson Crick base pairing between more than 85 % of the residues constituting the hybrid is required. In addition, a perfect complementarity over a segment of 10 constitutive nucleotide residues is required.

In the context of the present invention, the term "hybridizing adjacently" shall mean that in case the two hybridization probes are hybridized to the target nucleic acid, there exists either no or only a small gap ranging over 0-10 and preferably 1-2 complementary nucleotide residues between the two probes with respect to the target nucleic acid sequence

In another aspect, the invention is defined as being directed to a pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein due to the presence of the spacer entities, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probes.

In this context, the term "not substantially affected" shall mean that it is not possible with any conventional direct or indirect method known in the art to detect a quenching effect of more than 20% which is due to any G residue present in the target or the probes with regard to fluorescence emission of the FRET acceptor entity when the hybridization complex has been formed.

In other words, the intensity of fluorescence emission from the FRET acceptor entity of a pair of FRET hybridization probes according to the invention, when hybridized to its target sequence is detectably increased compared to the intensity of fluorescence emission of a FRET acceptor entity of a comparative pair of FRET hybridization probes hybridized to the same target DNA, if said comparative pair of FRET hybridization probes is identical to said pair of hybridization probes with the exception that said comparative pair of hybridization probes does not comprise a spacer entity connecting said nucleotide sequence entity and said fluorescent entity.

In this context, the term "detectably increased" shall mean that a difference between the two mentioned pairs of FRET hybridization probes can be monitored in a conventional real time PCR assay, for example a LightCycler instrument (Roche Applied Sciences). Preferably, however, the detectable difference is more than 20%.

The degree of fluorescence signal increase depends significantly on the target nucleic acid sequence and on the pair of FRET dyes used. In certain cases the fluorescence signal increase can exceed 100%. When using quenching-sensitive dyes, more than 5-fold signal increase can be observed when comparing a pair of FRET hybridization probes according to the invention with a pair of standard FRET hybridization probes. Fluorescence signal increase is not only observed in conventional hybridization asssays. As will be shown in the examples, it is also detectable in real time PCR quantification and in melting curve analysis.

In general, the nucleotide residues carrying the spacer linked to the fluorescent moiety in principle may be either internal, 5' terminal or 3' terminal residues, as long as upon hybridization of the pair of oligonucleotides to the target nucleic acid, fluorescence resonance energy transfer can take place to an extend, wherein both FRET entities are brought into spatial vicinity such that subsequent to excitation of the FRET donor, fluorescence emission from the FRET acceptor can be monitored.

Preferably, however, one oligonucleotide carrying the first spacer and the first FRET entity is labeled at its 3' terminal residue and the second oligonucleotide carrying the second spacer and the second FRET entity is labeled at its 5' terminal residue such that when both oligonucleotides are hybridized to the target nucleic acid, the fluorescent labels of both FRET entities are brought in close vicinity to each other due to the fact that said terminal residues are base pairing to adjacent residues in the target nucleic acid or at least to residues which are only separated by one, two or at maximum less then 10 further residues.

In this context, it may be chosen arbitrarily which oligonucleotide carries the FRET donor moiety and which oligonucleotide carries the FRET acceptor moiety, since these elements can be introduced on both, either the 5' or the 3' end of an oligonucleotide by methods known in the art.

Moreover, the present invention can also be defined as being directed to a pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
with the provisions that
- due to the presence of the spacer entities, the intensity of fluorescence emission from said FRET donor entity and the intensity of fluorescence emission from said FRET acceptor entity is not substantially affected by any quenching activity of nucleotide residues either present in the sequence of said target nucleic acid or present in said nucleotide sequence entities of said hybridization probes, and
- said spacer entities of the two members of said pair of FRET hybridization probes are capable of forming non covalent interactions with each other.

In a specific embodiment, the non covalent interactions between the two members of a pair of FRET hybridization probes according to the invention as disclosed above, are nucleotide base pairing interactions and preferably A/T base pairing interactions, forming a stem structure.

It has been proven to be advantageous, if the stem structure consists of two complementary single strands with equal numbers of nucleotide residues, however, it is also possible that the numbers of nucleotide residues is unequal, as long as upon hybridization, the two fluorescent moieties are brought in close vicinity to each other. This is usually the case, if the numbers of nucleotide residues only differ by 1 or 2. In addition, it is also within the scope of the present invention, if the stem structure formed by the nucleotides generating the base pairing interactions optionally comprises single mismatches and/or nucleotide analogues and a basic linkers.

In order to avoid any effect of quenching due to nucleotide residues like G residues, each stem should comprise at least 1-3 additional base pairs and preferably A/T base pairs. On the other hand, introduction of 10 or more additional nucleotide residues on each spacer entity does not result in any improved effect, but on the other hand may lead to an undesired complex formation between the two oligonucleotides without binding to the target nucleic acid itself.

The first oligonucleotide is designed in such a way that 3' to the nucleotide sequence entity which is substantially complementary to the sequence of the target nucleic acid, there are 1-10, preferably 3-8, and most preferably 3-5 additional nucleotide residues which act as a spacer entity. The 3' terminal residue of these additional residues is labeled with the first FRET entity according to standard protocols known in the art. Preferably, said additional residues are A or T residues. Highly prefered, more than 60 % of said additional residues are A or T residues. Also highly prefered, the two or even better the three 3' terminal residues are A or T residues.

Correspondingly, the second oligonucleotide is designed in such a way that 5' to the nucleotide sequence entity which is substantially complementary to the sequence of the target nucleic acid, there are 1-10, preferably 3-8, and most preferably 3-5 additional nucleotide residues which act as a spacer entity and at the same time are capable of hybridizing to the spacer entity of the first oligonucleotide. The 5' terminal residue of these additional residues is labeled with the second FRET entity again according to standard protocols known in the art. Preferably, said additional residues are A or T residues. Highly prefered, more than 60 % of said additional residues are A or T residues. Also highly prefered, the two or even better the three 3' terminal residues are A or T residues.

Moreover, it is also within the scope of the invention, if the stem generated by the base pairing interactions contains one, two, three or at least less than five additional G/C base pairs. Although in this case it may be possible that the respective G residues of the stem may result in a quenching effect, the degree of quenching on different FRET hybridization probes is becoming comparable. However, in order to obtain strong fluorescence signal intensities, it is highly preferred, if at least the one, two or even better three terminal base pairs forming the stem are A/T base pairs.

When both oligonucleotide probes according to the invention are hybridized to the target nucleic acid, the fluorescent labels of both FRET entities are brought into close vicinity to each other but on the other hand are still separated over a certain distance from any potential G residue within either the target nucleic acid or G residues within that part of the hybridization probes which is substantially complementary to the target nucleic acid sequence.

In general, the design if hybridization probes according to the invention is applicable to any combination of fluorescent compounds, between which fluorescent energy transfer may take place. Illustratory examples which are not at all limiting the invention are Fluorescein/Cy5 (Amersham), Fluorescein/LC-Red-640 (Roche Applied Science), Fluorescein/LC-Red-705 (Roche Applied Science), and Fluorescein/JA286 (EP 747 447).

It is also within the scope of the invention, if the FRET acceptor entity is a quencher moiety different from a fluorecent compound and consequently, decrease in fluorescence from the FRET donor moiety is monitored. Examples for quencher compounds which may be used in this regard are Dabcyl (Kreuzer, K. A., et al., Clin Chem 47 (2001) 486-90.) or so called Black Hole Quenchers (WO 01/86001)

Usually, the FRET hybridization probes are typical single stranded DNA molecules. Nevertheless, any kind of modification is possible. For example, the single stranded DNA may contain non natural bases such as 7-deaza-purine, diamino-purine or C-nucleotides. The single stranded DNA may also have a modified suger-phosphate backbone such as 2-O-Mehtyl, Phosphothioate, or anything similar.

The oligonucleotides acting as FRET hybridization probes may be labeled with the required fluorescent entity at any position by methods known in the art. For example, the oligonucleotides may be labeled internally at the nucleoside base or the phosphate moiety.

Preferably, however, one oligonucleotide is labeled at the 5' end and the second oligonucleotide is labeled at the 3' end. Which oligonucleotide carries the FRET donor moiety and which oligonucleotide carries the FRET acceptor moiety may be chosen arbitrarily in this regard. Usually, the 5' label may be introduced at the end of the oligonucleotide synthesis using an appropriate phosphoramidate carrying a fluorescent compound. Alternatively, after oligonucleotide synthesis, an oligonucleotide carrying a reactive amino group may be labeled with a fluorescent compound activated as an NHS ester. For the 3' labeling of oligonucleotides, commercially available controlled pore glass particles are used as a solid support for the start of a chemical oligonucleotide synthesis, which comprise a tri-functional spacer entity with a fluorescent compound.

The scope of the present invention is not limited to spacer entities which are composed of additional nucleotide residues. Examples for other non covalent interactions which may be applied for the present invention are all kinds of hydrogen bonding, for example polypeptide interactions, and all kinds of hydrophobic interactions, for example based on-CF₂ groups and ionic attractions. Thus, spacers with non covalent interactions involve all kinds of hydrogen bonding (like in peptides/ oligonucleotides) and all kinds hydrophobic interactions (like aryl-aryl, alkyl-alkyl interaction or attraction between fluorinated hydrocarbons). Ionic interaction can be used if one of the spacer is negatively charged and the other spacer is positively charged. In addition, it is possible that the spacer moiety is branched instead of being linear.

In another aspect, the present invention is also directed to compositions comprising the inventive FRET hybridization probes disclosed above. More precisely, a composition according to the invention comprises a nucleic sample and a pair of FRET hybridization probes, wherein said pair of FRET hybridization probes hybridizes adjacently to a target nucleic acid sequence, and each hybridization probe comprises (i) a nucleotide sequence entity which is substantially complementary to the sequence of the target nucleic acid, (ii) a fluorescent entity, said entity being either the FRET donor entity or the FRET acceptor entity, and (iii) a spacer entity connecting said nucleotide sequence entity and said fluorescent entity, wherein due to the presence of the spacer entity, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probes.

The present invention is also directed to various methods and applications of using the invenitve oligonucleotide pairs and compositions disclosed above. More precisely, the present invention is directed to a method for qualitative or quantitative detection of a nucleic acid sequence in a nucleic acid sample, wherein said nucleic acid sample is being hybridized with a pair of FRET hybridization probes wherein said pair of FRET hybridization probes hybridizes adjacently to a target nucleic acid sequence, and each hybridization probe comprises (i) a nucleotide sequence entity which is substantially complementary to the sequence of the target nucleic acid, (ii) a fluorescent entity, said entity being either the FRET donor entity or the FRET acceptor entity, and (iii) a spacer entity connecting said nucleotide sequence entity and said fluorescent entity, wherein due to the presence of the spacer entity, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probe.

In one embodiment, such a method may be a typical hybridization assay. The hybridization may take place either in solution, or alternatively, either the target nucleic acid or one member of the pair of FRET hybridization probes already by immobilized on a solid support. The solid support itself, for example can be a hybridization membrane, a magnetic glass bead, a micro-array for immobilizing nucleic acids or any other material known in the art.

In another, preferred embodiment, a part of said nucleic acid present in the sample is being subjected to a nucleic acid amplification reaction prior or during the hybridization procedure, for example, a polymerase chain reaction (PCR). As a prerequisite, the target nucleic acid comprises a sequence substantially complementary or homologous to the sequence of the used hybridization probes. In other words, the used hybridization probes need to hybridize specifically to the part of the target nucleic acid, which is being amplified.

In addition, the invention provides a method, wherein a pair of FRET hybridization probes according to the invention is being used for monitoring the amplification of a target nucleic in real time. As it is known in the art, real time monitoring allows the generation of kinetic data and facilitates quantitative analysis. Thus, the present invention is also directed to a method of monitoring the amplification of a target nucleic acid by means of monitoring either the increase in fluorescence emission of the FRET acceptor entity or monitoring the decrease in fluorescence emission of the FRET donor entity during the amplification reaction itself.

In a further aspect, the present invention is directed to the usage of the FRET hybridization probes disclosed above for melting curve analysis, wherein monitoring of the dissociation of a complex between a target nucleic acid and a hybridization probe allows for the detection of small sequence variants such as single nucleotide polymorphisms.

More precisely, the invention is directed to a method for the determination of the melting profile of a hybrid consisting of a target nucleic acid and a pair of FRET hybridization probes according to the invention, characterized in that first, a ternary hybrid complex between the target nucleic acid and the two hybridization probes is formed. Subsequently, the temperature is increased and the thermal dissociation of the ternary complex is determined by means of monitoring fluorescence in real time. In other words, the new invention is also directed to a method for the determination of the melting profile of a hybrid consisting of a target nucleic acid and a pair of FRET hybridization probes as disclosed above, characterized in that the fluorescence emission is determined as a function of temperature.

In addition, it is emphasized that the design of the non covalent interactions between the two FRET hybridization probes and especially the selection of the number of A/T base pairing interactions may be used in order to obtain a pair of FRET hybridization probes with a distinct melting temperature. This is highly advantageous for the development of a multiplex assay comprising multiple pairs of hybridization probes in order to generate melting peaks which can unambigously be discriminated from each other.

In a last aspect, the present invention is directed to a kit comprising a pair of hybridization probes according to the invention. Such a kit may comprise a pair of FRET hybridization probes according to the invention as disclosed above. In addition, it may also contain oligonucleotides capable of acting as a primer pair for a nucleic acid amplification reaction.

Precisely, the kit comprises a pair of FRET hybridization probes consisting of a first oligonucleotide carrying a FRET donor entity and a second oligonucleotide carrying a FRET acceptor entity, wherein said pair of FRET hybridization probes hybridizes adjacently to a target nucleic acid sequence, and each hybridization probe comprises (i) a nucleotide sequence entity which is substantially complementary to the sequence of the target nucleic acid, (ii) a fluorescent entity, said entity being either the FRET donor entity or the FRET acceptor entity, and (iii) a spacer entity connecting said nucleotide sequence entity and said fluorescent entity, wherein due to the presence of the spacer entity, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probes.

In addition, a kit according to the present invention may contain at least one additional component such as a nucleic acid polymerase, deoxynucleoside triphosphates or respective analogues and an appropriate buffer which may be used for a template dependent nucleic acid amplification reaction such as PCR. Furthermore, the kit may also comprise software tools such as compact discs carrying computer programs for quantitative analysis of relative or absolute nucleic acid quantification experiments.

The following examples, references, sequence listing and figures are provided to aid the understanding of the present invention, the true scope of which is set forth in the appended claims. It is understood that modifications can be made in the procedures set forth without departing from the spirit of the invention.

### Example 1

### Preparation of PCR primers and probes

Primers were synthesized on a 1 µmol scale on ABI 394 synthesizer using commercially available standard phosphoramidites ( DMTr ibu G, DMTr bzA; DMTr bz C and DMTr T) and the corresponding CPG support. The chemicals for standard synthesis were obtained from GlenResearch. Removal of the oligonucleotides from the solid support and deprotection was carried out with 33 % NH₃ for 8h at 55 °C. Synthesis was performed in the trityl on modus. Purification was done on a RP 18 Oligo R3 4.6 x 50 mm column from Perseptive Biosystems) Buffer A: 0.1M Triethylammonium acetate in water pH 7.0 /MeCN 95:5 buffer B: MeCN. gradient 3 min 20 % B; 12 min 12- 40 % B flow rate 1 ml/min detection 260 nm. Subsequently, the concentrated oligonucleotide solution was treated for 5 min with 80 % Acetic acid at room temperature in order to remove the 5' DMTr protecting group. Afterwards, oligonucleotides were desalted with a RP 18 coluomn and lyophilyzed in a Speed Vac.

5' labeled Oligonucleotide (JA 286/ LC Red 640) synthesis was performed in the 1 µmol range. Commercially available standard phosphoramidites ( DMTr ibu G, DMTr bzA; DMTr bz C and DMTr T) and chemicals for standard synthesis were obtained from Glen Research. The 5' amino group was introducd by using commercially available 5' amino modifier (Glen Research (cat no. 10-1916-90) As solid support 3' phosphate CPG ( GlenResearch 20-2900-01) was used. Removal of the oligonucleotides from the solid support and deprotection was carried out with 33 % NH₃ for 8h at 55 °C. The solution was evaporated under vacuum. The remainder was dissolved in 600 µl double destilled water and transferred in a microcentrifuge tube 60 µl of sodium acetate buffer (3M, ph 8.5 were added). Upon addition of 1.8 ml ice cold ethanol the mixture was stored at -15 °C for 3 h. The solution was centrifuged at 10000 x g for 15 min. The supernatant was decanted. The pellet is washed with 200 µl ice cold ethanol. After centrifugation the supernatent was decanted. The pellet was dissolved in 400 µl sodium borate buffer (0.1M pH 8.5) and was labeled according standard procedures.

NHS-activated LC-Red-640 and JA286 were used. NHS-LC-Red-640 is obtainable from Roche applied Sciene (Cat. No: 2 015 161). NHS activated JA286 was synthesized according to EP 0747 447, example 1.

A solution of 1 mg of the dye NHS ester in DMF was added and reacted for 15 h The labeled oligonucleotide was purified by reversed phase using a Oligo R3 4.6 x 50 mm column) Chromatography: buffer A: 0.1M Triethylammoniamacetat in water pH 7.0 buffer B: 0.1 M triethylammonium acetate in water/MeCN 1:1. gradient 2 min 0 % B in 45 min to 100 %B ( the gradient was stopped when a product starts to eluate at 20 -25 % B the nonlabeled oligonucloetide eluates; at 60 -65 % B the desired labeld oligonucleotide eluates at 100 % B the dye eluates; flow rate was 1 ml/min detection at 260 nm. The fractions from the labeled oligonucloetide peaks were collected and the solvent was removed by using a vaccum centrifuge. The remainder was dissolved in double distilled water and then evaporated again with vaccuum centrifuge, This procedure was repeated three times. The pellet was dissolved in water and lyophilized.

3' Fluorescein labeled oligonucleotides were synthesized and purified according to the pack insert of the commercially available LightCycler Fluorescein CPG (Roche Applied Sience cat no. 3138178)

### Example 2

### Quantitative Real time PCR of Factor V DNA using a pair of FRET hybridization probes labeled with Fluorescein/ JA286

For amplification of a Factor V DNA fragment, a 20µl Real Time PCR reaction mixtures was set up as follows:

| | |
|---|---|
| 10⁶ or 10⁴ | copies of a plasmid containing the Factor V gene (Gene Bank Accession No: M_014335 ) |
| 13 mM | MgCl₂ |
| | |
| 500 nM | each primers according to SEQ. ID. NO: 1 and 2 |
| 200 nM | each FRET hybridization probes according to SEQ.ID. NO: 3 and 4 or 5 and 6, respectively. |

PCR components of LightCycler DNA Master Hyb Probes Kit (Roche Applied Science, Cat. No. 2158825)

Primers and probes were used as follows:

SEQ ID NO:1
Forward primer:
5′ GAG AGA CAT CGC CTC TGG GCT A

SEQ ID NO:2
Reverse Primer
5′ TGT TAT CAC ACT GGT GCT AA

SEQ ID NO:3
FRET donor probe
5′ AAT ACC TGT ATT CCT CGC CTG TC-Fluorescein

SEQ ID NO:4
FRET acceptor probe
5′ JA286-AGG GAT CTG CTC TTA CAG ATT AGA AGT AGT CCT ATT

SEQ ID NO:5
FRET donor probe
5′ AAT ACC TGT ATT CCT CGC CTG TCA AAA A-Fluorescein

SEQ ID NO:6
FRET acceptor probe
5' JA286-TTT  TTA  GGG ATC TGC TCT TAC AGA TTA GAA GTA GTC CTA TT

The probes according to SEQ.ID.NO: 3 and 5 were 3' terminally labeled with Fluorescein according to example 1. The probes according to SEQ. ID. NO: 4 and 6 were 5' terminally labeled with JA286 as a FRET acceptor according to example 1. Since SEQ. Id. No: 5 comprises a 3' terminal oligo-A pentamer, and SEQ.ID. NO: 6 comprises a 5'terminal oligo-T pentamer, a respective FRET hybridization probe pair constitutes a pair of hybridzation probes according to the invention.

Amplification was performed in a LightCycler instrument (Roche Applied Science) according to the following thermocycling protocol:

**Table 1:**

| | T[°C] | t[sec] | Ramp-rate[°C/sec] | Acquisition | Cycles |
|---|---|---|---|---|---|
| Denaturation | 95 | 30 | 20.0 | none | 1 |
| Amplification | 95 | 0 | 20.0 | none | |
| | 55 | 10 | 20.0 | single | 45 |
| | 72 | 10 | 20.0 | none | |

Real time monitoring was performed using the 2^{nd} derivative threshold method over 45 cycles by measuring the fluorescence signals in a detection channel specific for JA286 emission (at 710 nm) and using arithmetic background correction for normalization of initial fluorescence background intensities..

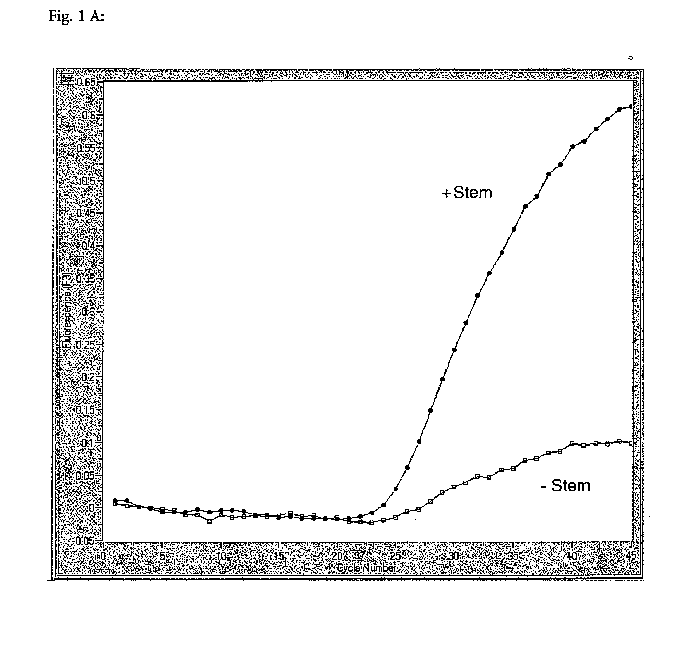

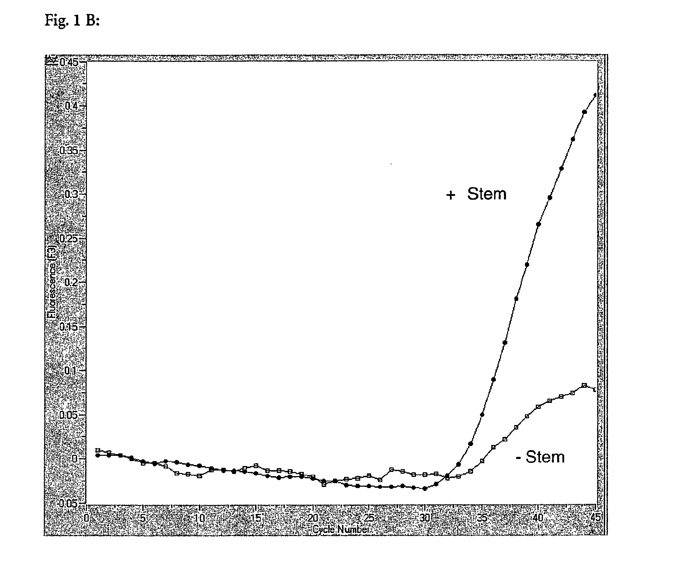

The result is shown in fig. 1. As can be seen in the figure, for both copy numbers of target DNA tested (fig.1a: 10⁶ copies, fig. 1b: 10⁴ copies), usage of FRET hybridization probes comprising an AT stem according to the invention resulted in significantly increased amplification signal.

### Example 3

### Melting Curve Analysis of Factor V DNA using a pair of FRET hybridization probes labeled with Fluorescein/ JA286

Subsequent to the reaction disclosed in example 2, the samples were subjected to a melting curve analysis according to the instructions of the LightCycler manual (Roche Applied Sciences) using the following temperature transition protocol:

**Table 2:**

| | T[°C] | t[sec] | Ramp-rate[°C/sec] | Acquisition | Cycles |
|---|---|---|---|---|---|
| Melting curve | 95 | 0 | 20.0 | none | |
| | 45 | 60 | 20.0 | continous | 1 |
| | 75 | 10 | 0.1 | none | |
| Cooling | 40 | 30 | 20.0 | none | 1 |

Fluorescence monitoring was performed by measuring the absolute signal values obtained in the JA286 channel at 710 nm and subsequent calculation of the first derivative.

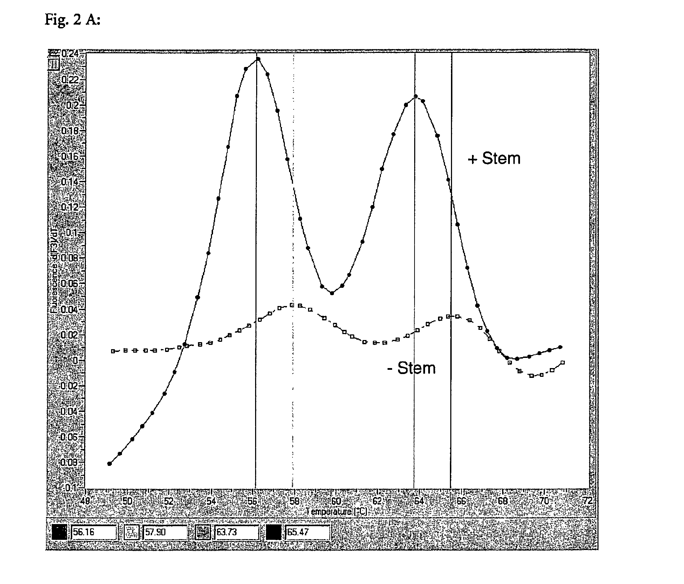

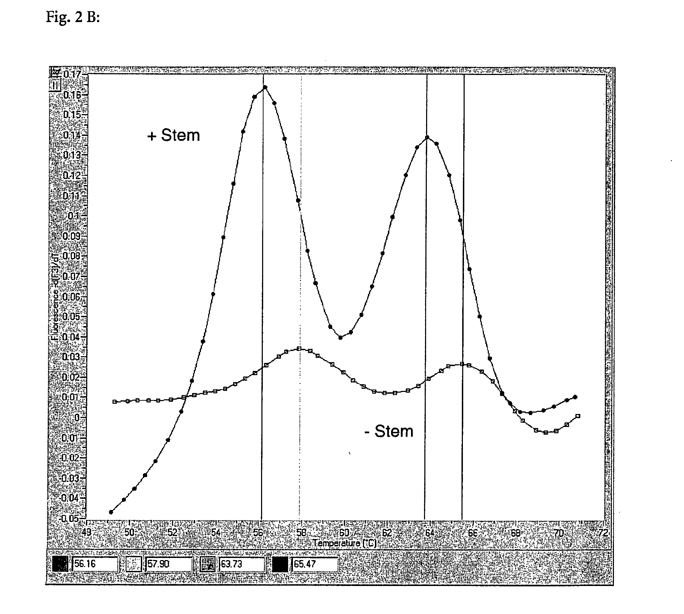

The result is shown in fig. 2. As can be seen in the figure, for both copy numbers of target DNA tested (fig.2a: 10⁶ copies, fig. 2b: 10⁴ copies), usage of FRET hybridization probes comprising an AT stem according to the invention resulted in significantly increased melting peaks.

### Example 4

### Quantitative Real time PCR of Factor V DNA using a pair of FRET hybridization probes labeled with Fluorescein/ LC-Red-640

Amplification of a Factor V DNA fragment was performed as in example 2 with the exception that instead of JA286, LC-Red 640 was used as FRET acceptor moiety for 5' terminal labeling of the hybridization probe according to SEQ.ID.NO: 4 and 6:

SEQ ID NO:1
Forward primer:
5' GAG AGA CAT CGC CTC TGG GCT A

SEQ ID NO:2
Reverse Primer
5′ TGT TAT CAC ACT GGT GCT AA

SEQ ID NO:3
FRET donor probe
5′ AAT ACC TGT ATT CCT CGC CTG TC-Fluorescein

SEQ ID NO:4
FRET acceptor probe
5′ LCRED640-AGG GAT CTG CTC TTA CAG ATT AGA AGT AGT CCT ATT

SEQ ID NO:5
FRET donor probe
5′ AAT ACC TGT ATT CCT CGC CTG TCA AAA A-Fluorescein

SEQ ID NO:6

FRET acceptor probe
5′ LCRED640-TTT TTA GGG ATC TGC TCT TAC AGA TTA GAA GTA GTC CTA TT

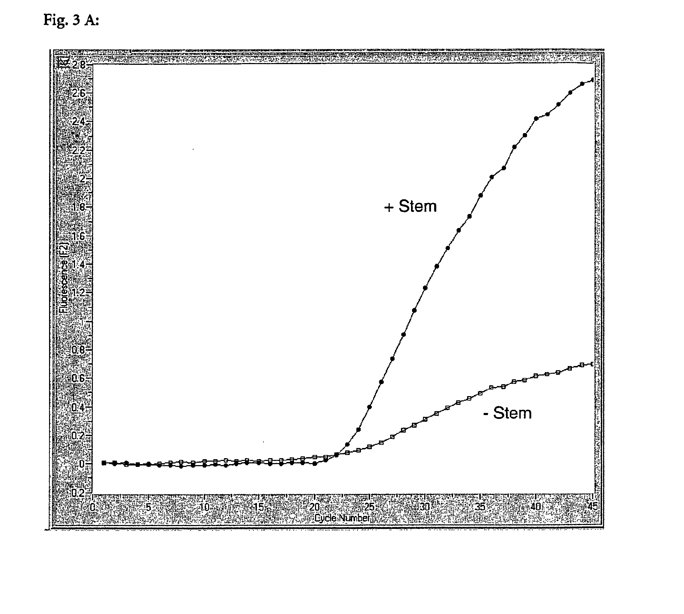

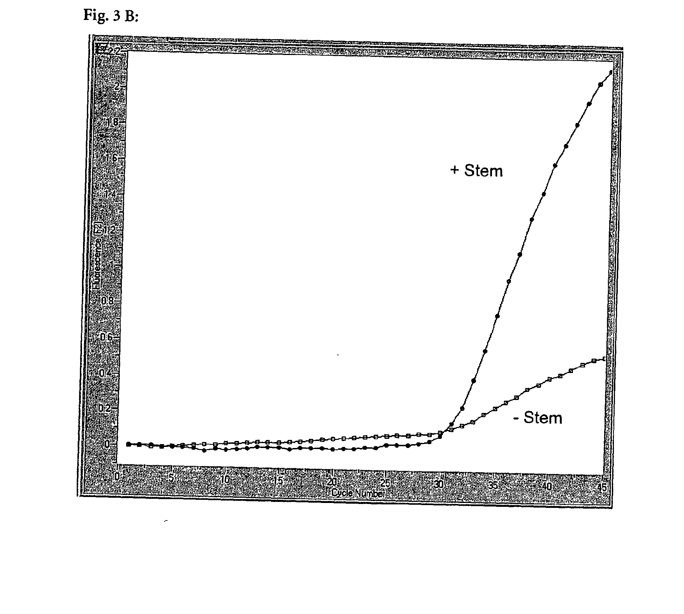

The result is shown in fig. 3. As can be seen in the figure, for both copy numbers of target DNA tested (fig.3a: 10⁶ copies, fig. 3b: 10⁴ copies), the effect of an increased amplification signal using FRET hybridization probes comprising an AT stem according to the invention (by using a FRET pair according to Seq. Id. Nos: 5 and 6) could also be observed with a different FRET pair. It can be concluded that the positive effect of the claimed invention is independent from the type of dye pair which is actually used.

### Example 5

### Melting Curve Analysis of Factor V DNA using a pair of FRET hybridization probes labeled with Fluorescein/ LC-Red-640

Subsequent to the reaction disclosed in example 4, the samples were subjected to a melting curve analysis identical to the conditions disclosed in example 3.

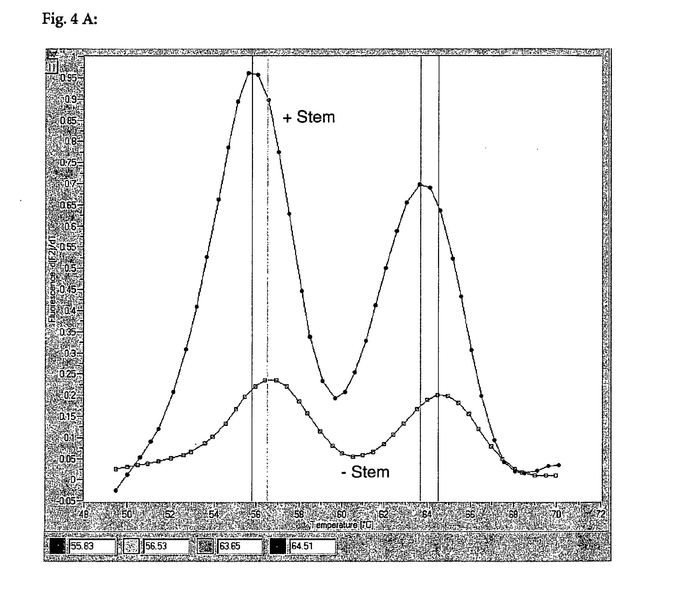

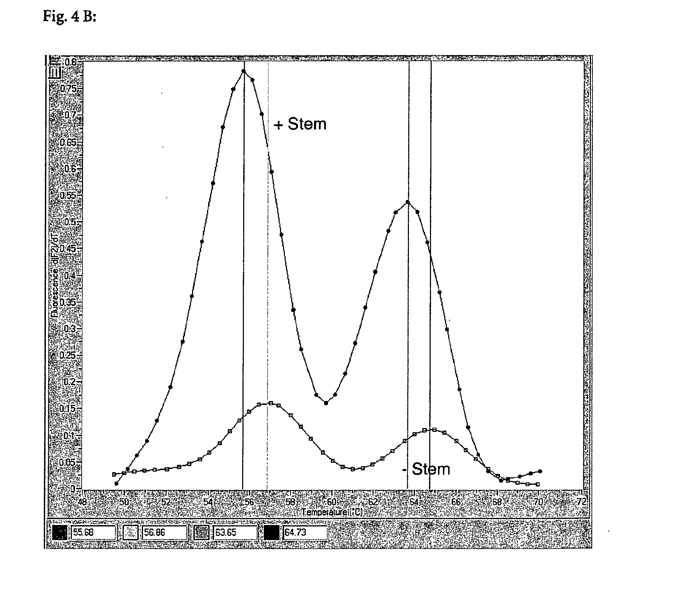

The result is shown in fig. 4. As can be seen in the figure, for both copy numbers of target DNA tested (fig.4a: 10⁶ copies, fig. 4b: 10⁴ copies), usage of a FRET hybridization probes comprising an AT stem according to the invention again resulted in a significant increase of melting peaks. Thus it can be concluded that improved melting curve analysis according to the invention is independent from the type of dye pairs used.

### Example 6

### Quantitative Real time PCR of G6PDH DNA using a pair of FRET hybridization probes labeled with Fluorescein/ JA286

For amplification of the G6PDH DNA (Gene Bank Acc. No: XM_013149 ) fragment, conditions were identical as disclosed in example 2, amplifying 10⁴ copies of target DNA with the following primers and probes:

SEQ ID NO:7
Forward primer
GGG TGC ATC GGG TGA CCT G

SEQ ID NO:8
Reverse Primer
AGC CAC TGT GAG GCG GGA

SEQ ID NO:9
FRET donor probe

5′ GGT GTT TTC GGG CAG AAG GCC ATC C-Fluorescein

SEQ ID NO:10
FRET acceptor probe
5′ JA286-AAC AGC CAC CAG ATG GTG GGG TAG ATC TT

SEQ ID NO:11
FRET donor probe
5′ GGT GTT TTC GGG CAG AAG GCC ATC CAA AAA-Fluorescein

SEQ ID NO:12
FRET acceptor probe
5′ JA286-TTT TTA ACA GCC ACC AGA TGG TGG GGT AGA TCT T

In particular, 500 nM primers (each) according to SEQ. ID. NO: 7 and 8, and 200 nM FRET hybridization probes (each) according to SEQ.ID. NO: 9 and 11 or 10 and 12 repectively. were used.

The probes according to SEQ.ID.NO: 9 and 11 were 3' terminally labeled with Fluorescein according to example 1. The probes according to SEQ. ID. NO: 10 and 12 were 5'terminally labeled with JA286. Since SEQ. Id. No: 11 comprises a 3' terminal oligo-A pentamer, and SEQ.ID. NO: 12 comprises a 5'terminal oligo-T pentamer, a respective FRET hybridization probe pair constitutes a pair of hybridization probes according to the invention.

Amplification was performed in a LightCycler instrument (Roche Applied Science) according to the following thermocycling protocol:

**Table 3:**

| | T[°C] | t[sec] | Ramp-rate[°C/sec] | Acquisition | Cycles |
|---|---|---|---|---|---|
| Denaturation | 95 | 60 | 20.0 | none | 1 |
| Amplification | 95 | 0 | 20.0 | none | |
| | 55 | 15 | 20.0 | single | 45 |
| | 72 | 15 | 20.0 | none | |
| Cooling | 40 | 30 | 20.0 | none | 1 |

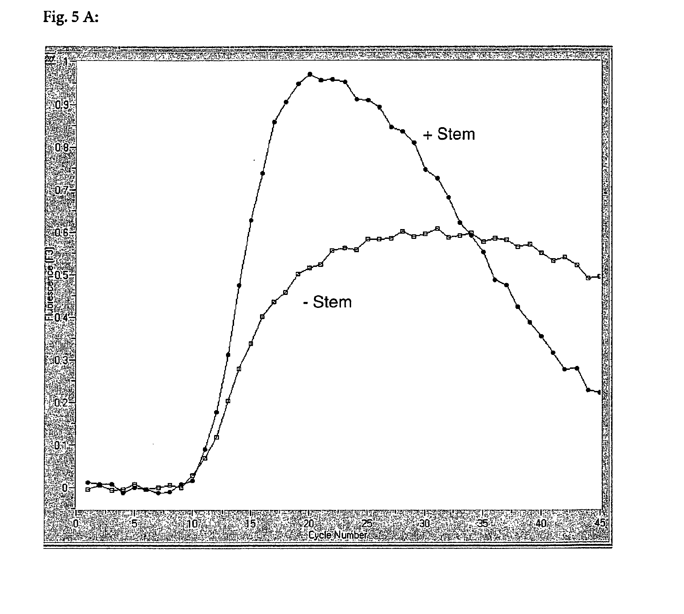

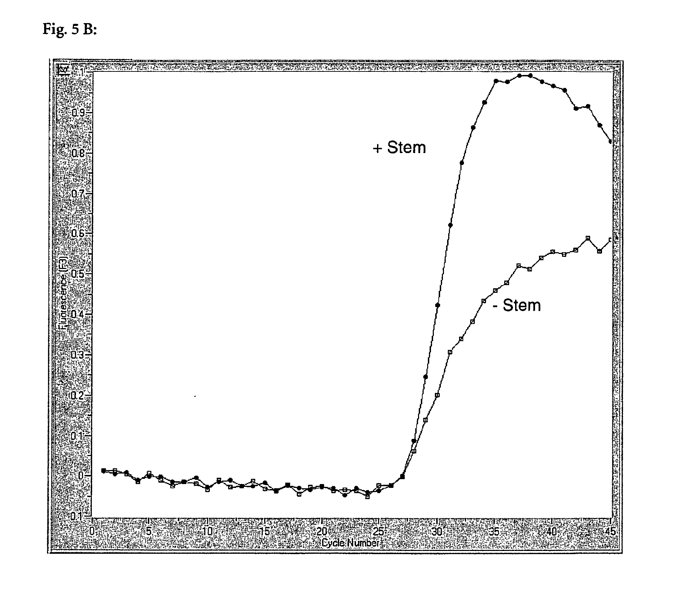

The result is shown in fig. 5. As can be seen in the figure, for both copy numbers of target DNA tested (fig.5a: 10⁸ copies, fig. 5b: 10³ copies), usage of a FRET hybridization probes comprising an AT stem according to the invention also resulted in a significantly increased amplification signal when another target DNA fragment was amplified. Thus it can be concluded that the new invention provides FRET hybridization probes which confer increased amplification signals independent from the type of target DNA to be amplified.

### Example 7

### Quantitative real time PCR of Factor V DNA using FRET hybridization probes labeled with Fluorescein/ JA286 comprising different A/T stems

The experiment was performed as disclosed in example 2 with the modification that hybridization probes having no, one, three or five A/T base pair stems were tested.

Thus, primers and probes were used as follows:

SEQ ID NO:1
Forward primer:
5′ GAG AGA CAT CGC CTC TGG GCT A

SEQ ID NO:2
Reverse Primer
5′ TGT TAT CAC ACT GGT GCT AA

SEQ ID NO:3
FRET donor probe
5′ AAT ACC TGT ATT CCT CGC CTG TC-Fluorescein

SEQ ID NO:4
FRET acceptor probe
5′ JA286-AGG GAT CTG CTC TTA CAG ATT AGA AGT AGT CCT ATT

SEQ ID NO:13
FRET donor probe 1A
5′ AAT ACC TGT ATT CCT CGC CTG TCA-Fluorescein

SEQ ID NO:14
FRET acceptor probe 1T
5′ JA286-TAG GGA TCT GCT CTT ACA GAT TAG AAG TAG TCC TAT T

SEQ ID NO:15
FRET donor probe 3A
5′ AAT ACC TGT ATT CCT CGC CTG TCA AA-Fluorescein

SEQ ID NO:16
FRET acceptor probe 3T
5′ JA286-TTT AGG GAT CTG CTC TTA CAG ATT AGA AGT AGT CCT ATT

SEQ ID NO:5
FRET donor probe 5A
5′ AAT ACC TGT ATT CCT CGC CTG TCA AAA A-Fluorescein

SEQ ID NO:6
FRET acceptor probe 5T
5′ JA286-TTT  TTA  GGG ATC TGC TCT TAC AGA TTA GAA GTA GTC CTA TT

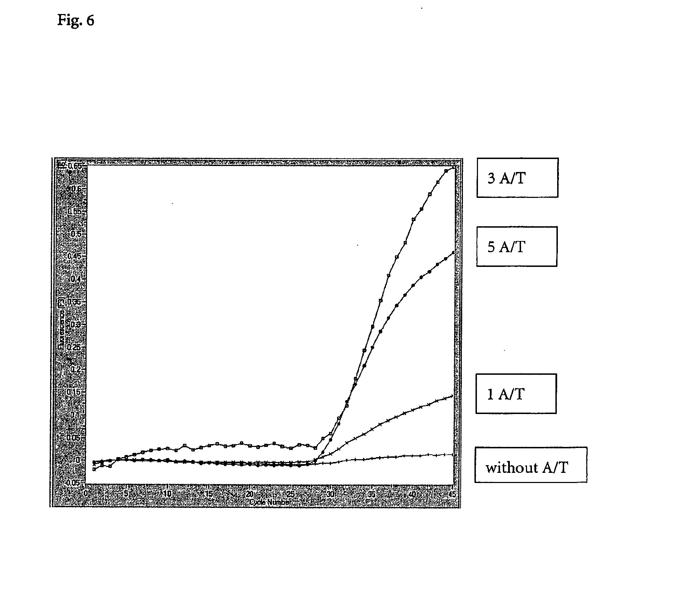

The result is shown in fig. 6. As can be seen in the figure, an improved amplification signal was already obtained with an A/T stem consisting of only one A/T base pair, the nucleotide residues of which do not hybridize to the target DNA. Moreover, the effect was significantly increased with A/T stems consisting of 3 or 5 A/T stems. Thus, it seems that the optimum length of an A/T stem according to the invention is between 3-5 base pairs.

### Example 8

### Melting Curve Analysis of Factor V DNA using hybridization probes labeled with Fluorescein/ JA286 comprising different A/T stems

Subsequent to the reaction disclosed in example 7, the samples were subjected to a melting curve analysis as disclosed in example 3.

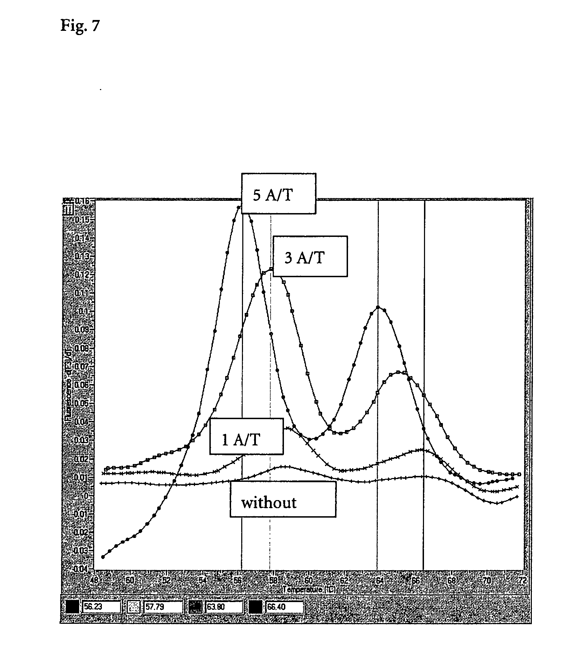

The result is shown in fig. 7. As can be seen in the figure, all FRET hybridization probes comprising an AT stem according to the invention resulted in significantly increased melting peaks, even if the A/T stem was consisting of only one base pair.

### List of References

Bernard, P. S., et al., Anal Biochem 255 ( 1998) 101-7.
Kreuzer, K. A., et al., Clin Chem 47 (2001) 486-90.
Seidel, C. A. M., et al., J Phys Chem 100 (1996) 5541-53
EP0747447
EP1046717
WO0136668
WO0173118
WO0186001
WO9746707
WO9746712
WO9746714

## Claims

1. A pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein said spacer entities of the two members of said pair of FRET hybridization probes are capable of forming non covalent interactions with each other.

2. A pair of FRET hybridization probes according to claim 1, wherein said non covalent interactions are nucleotide base pairing interactions and preferably A/T base pairing interactions.

3. A pair of FRET hybridization probes hybridizing adjacently to a target nucleic acid sequence, each hybridization probe comprising
- a nucleotide sequence entity which is substantially complementary to the sequence of the target nucleic acid
- a fluorescent entity, said entity being either the FRET donor entity or the FRET acceptor entity
- a spacer entity connecting said nucleotide sequence entity and said fluorescent entity,
wherein due to the presence of the spacer entities, the intensity of fluorescence emission from said FRET donor entity and the intensity of fluorescence emission from said FRET acceptor entity are not substantially affected by any quenching activity of nucleotide residues either present in the sequence of said target nucleic acid or present in said nucleotide sequence entities of said hybridization probes.

4. A pair of FRET hybridization probes according to claim 3, wherein the intensity of fluorescence emission from said FRET acceptor entity when hybridized to its target sequence is detectably increased compared to the intensity of fluorescence emission of a FRET acceptor entity of a comparative pair of FRET hybridization probes hybridized to the same target DNA,
said comparative pair of FRET hybridization probes being identical to said pair of hybridization probes with the exception that said comparative pair of hybridization probes does not comprise a spacer entity connecting said nucleotide sequence entity and said fluorescent entity.

5. A composition comprising a nucleic acid sample and a pair of hybridization probes according to claims 1-4.

6. A kit comprising a pair of hybridization probes according to claims 1-4 and at least one other component selected from a group consisting of nucleic acid amplification primers, template dependent nucleic acid polymerase, deoxynucleoside triphosphates and a buffer for template dependent nucleic acid amplification reaction.

7. Method for qualitative or quantitative detection of a nucleic acid sequence in a biological sample, wherein the nucleic acid present in said sample is hybridized with a pair of FRET hybridization probes according to claim 1-4.

8. Method according to claim 7, wherein a part of said nucleic acid present in said sample, which comprises a target nucleic acid sequence substantially complementary to the sequences of said pair of FRET hybridization probes is amplified by a nucleic acid amplification reaction, in particular by a polymerase chain reaction.

9. Method according to claim 8, wherein fluorescence emission of either the FRET donor entity or emission of the FRET acceptor entity is monitored in real time.

10. Method for the determination of the melting profile of a hybrid consisting of a target nucleic acid and a pair of FRET hybridization probes according to claims 1-4,
**characterized in that** the fluorescence emission is determined as a function of temperature.


SEQUENCE LISTING

<110> Roche Diagnostics GmbH
F. Hoffmann-La Roche AG

<120> Improved Fluorescent Resonance Energy Transfer Probes

<130> 21339 EP

<140>
<141>

<160> 16

<170> PatentIn Ver. 2.1

<210> 1
<211> 22
<212> DNA
<213> Artificial Sequence

<220>
<223> Description of Artificial Sequence:primer

<400> 1
gagagacatc gcctctgggc ta 22

<210> 2
<211> 20
<212> DNA
<213> Artificial Sequence

<220>
<223> Description of Artificial Sequence:primer

<400> 2
tgttatcaca ctggtgctaa 20

<210> 3
<211> 23
<212> DNA
<213> Artificial Sequence

<220>
<223> Description of Artificial Sequence:primer

<400> 3
aatacctgta ttcctcgcct gtc 23

<210> 4
<211> 36
<212> DNA
<213> Artificial Sequence

<220>
<223> Description of Artificial Sequence:primer

<400> 4
agggatctgc tcttacagat tagaagtagt cctatt 36

<210> 5
<211> 28
<212> DNA
<213> Artificial Sequence

<220>
<223> Description of Artificial Sequence:primer

<400> 5
aatacctgta ttcctcgcct gtcaaaaa 28

<210> 6
<211> 41
<212> DNA
<213> Artificial Sequence

<220>
<223> Description of Artificial Sequence:primer

<400> 6
tttttaggga tctgctctta cagattagaa gtagtcctat t 41

<210> 7
<211> 19
<212> DNA
<213> Artificial Sequence

<220>
<223> Description of Artificial Sequence:primer

<400> 7
gggtgcatcg ggtgacctg 19

<210> 8
<211> 18
<212> DNA
<213> Artificial Sequence

<220>
<223> Description of Artificial Sequence:primer

<400> 8
agccactgtg aggcggga 18

<210> 9

<211> 25
<212> DNA
<213> Artificial Sequence

<220>
<223> Description of Artificial Sequence:primer

<400> 9
ggtgttttcg ggcagaaggc catcc                                25

<210> 10
<211> 29
<212> DNA
<213> Artificial Sequence

<220>
<223> Description of Artificial Sequence:primer

<400> 10
aacagccacc agatggtggg gtagatctt                            29

<210> 11
<211> 30
<212> DNA
<213> Artificial Sequence

<220>
<223> Description of Artificial Sequence:primer

<400> 11
ggtgttttcg ggcagaaggc catccaaaaa                           30

<210> 12
<211> 34
<212> DNA
<213> Artificial Sequence

<220>
<223> Description of Artificial Sequence:primer

<400> 12
tttttaacag ccaccagatg gtggggtaga tctt                      34

<210> 13
<211> 24
<212> DNA
<213> Artificial Sequence

<220>
<223> Description of Artificial Sequence:primer

<400> 13

aatacctgta ttcctcgcct gtca 24

<210> 14
<211> 37
<212> DNA
<213> Artificial Sequence

<220>
<223> Description of Artificial Sequence:primer

<400> 14
tagggatctg ctcttacaga ttagaagtag tcctatt 37

<210> 15
<211> 26
<212> DNA
<213> Artificial Sequence

<220>
<223> Description of Artificial Sequence:primer

<400> 15
aatacctgta ttcctcgcct gtcaaa 26

<210> 16
<211> 39
<212> DNA
<213> Artificial Sequence

<220>
<223> Description of Artificial Sequence:primer

<400> 16
tttagggatc tgctcttaca gattagaagt agtcctatt 39